# EUROPÄISCHE PATENTSCHRIFT

(11) **EP 0 701 417 B1**
(45) Veröffentlichungstag und Bekanntmachung des Hinweises auf die Patenterteilung: **19.08.1998**
(21) Anmeldenummer: 94915043.7
(22) Anmeldetag: 19.05.1994
(51) Int. Cl.: A61B 17/10, A61L 31/00

(54) **ANASTOMOSEVORRICHTUNG**
ANASTOMOSIS DEVICE
DISPOSITIF D'ANASTOMOSE

(30) Priorität: 21.05.1993 DE 4317103
(43) Veröffentlichungstag der Anmeldung: 20.03.1996
(73) Patentinhaber: BIOVISION GmbH Entwicklung, Herstellung und Vertrieb von Biomaterialien, D-98693 Ilmenau (DE)
(72) Erfinder: BÄHR, Wolfgang, Universitätsklinik, D-79106 Freiburg (DE); KÖPFER, Simon, D-79268 Bötzingen (DE); SCHOLZ, Christian, D-79224 Umkirch (DE); REIF, Dieter, D-98529 Suhl (DE); HUTMACHER, Dietmar, D-79100 Freiburg (DE)
(74) Vertreter: Liedtke, Klaus, Dr.
(86) Internationale Anmeldenummer: DE9400577
(87) Internationale Veröffentlichungsnummer: WO9427506

(56) Entgegenhaltungen:
- EP-A- 0 154 103
- EP-A- 0 202 444
- EP-A- 0 316 992
- WO-A-92/13579
- DE-A- 4 118 201
- GB-A- 2 000 684
- US-A- 3 683 926
- US-A- 4 470 415

## Beschreibung

Die Erfindung betrifft eine Anastomosevorrichtung, bestehend aus Trägerhülsen aus resorbierbarem biokompatiblem Werkstoff für die Gewebeenden und aus einem diese verbindenden schrumpffähigen, rohrförmigen Hohlkörper aus biokompatiblem Werkstoff.

In der Gefäßchirurgie ist bisher die Nahttechnik die am weitesten verbreitete Methode zum Verbinden der Enden zweier Blutgefäße. Diese Methode ist einerseits relativ zeitaufwendig, andererseits beinhaltet sie durch die damit verbundene Gefäßschädigung eine gewisse Gefahr der Thrombusbildung.

Es wurde deshalb frühzeitig versucht, diesem Nachteil zu begegnen und Verfahren und Vorrichtungen zu entwickeln, die eine Verbindung ohne Nahttechnik ermöglichen. Eine Übersicht über die wichtigsten Entwicklungsetappen nach Östrup und Berggren, Annals of Plastic Surgery, Vol. 17, No 6, Dec. 1986, Seite 522, zeigt Tabelle 1.

In "Plastic and Reconstructiv Surgery", September 1984, Seite 329, wird eine resorbierbare Vorrichtung zur Anastomose nach Daniel beschrieben, die sich der Hülsenkupplungstechnik bedient. Die Vorrichtung besteht aus zwei Hülsen und einer Kupplung. Die Blutgefäßenden werden durch die Hülsen gezogen und umgestülpt. Die beiden Hülsen werden dann in die Kupplungsmuffe geschoben und rasten so ein, daß ein optimaler Kontakt der beiden Gefäßenden gewährleistet ist. Das System aus resorbierbarem Polymer wird innerhalb von 70 Tagen hydrolytisch abgebaut.

Ein nicht resorbierbares Instrumentensystem für die schnelle und sichere Mikrogefäß-Anastomose (UNILINK) wird in "Annals of Plastic Surgery, Vol. 17, No. 6, Dec. 1986, Seite 522-525, beschrieben. Dieses System bedient sich der Ring-Pin-Technik.

Es besteht aus zwei gleichen Ringen, die an der Stirnseite jeweils 6 Pins und 6 Löcher so angeordnet enthalten, daß die Pins in die Löcher des Gegenringes einrasten. Die Blutgefäßenden werden durch jeweils einen Ring gezogen und an den Pins fixiert. Die beiden Ringe werden durch eine Ring-Pin-Haltevorrichtung zusammengeklinkt und so die Anastomose durch das gegenseitige Andrücken der Innenseiten der beiden Gefäßenden realisiert.

Einfache chirurgische Clips, die auch in der Gefäßanastomose zur Anwendung kommen können, werden in der DE 34 43 367 und der WO 90/10418 beschrieben. Aus Metallen gefertigt, besitzen diese den Nachteil, daß Fremdkörper im Organismus verbleiben. Dieser Nachteil wird zwar durch den Einsatz resorbierbarer Polymere aufgehoben, ein hoher Platzbedarf setzt der Anwendung zu geringeren Gefäßdurchmessern jedoch Grenzen. Ein Restrisiko bezüglich Undichtigkeit zwischen den einzelnen gesetzten Clips bleibt erhalten.

**Tabelle 1**

| Übersicht über bisher entwickelte Anastomose-Systeme in der Mikrochirurgie, mechanische nahtfreie Anastomose | | | | | | | |
|---|---|---|---|---|---|---|---|
| *Jahr* | *Entstehungsland* | *Erfinder* | *Marktname* | *technisches Prinzip* | *Anastomosetyp* | *Lageveränderung der Gefäßwand (Grad)* | *minimaler Gefäßdurchmesser (mm)* |
| 1956 | UdSSR | Androsov | | Stapler | End-to-end End-to-side | 180 | 2,0 |
| 1958 | Japan | Inokuchi | | Stapler | End-to-end End-to-side | 180 | 2,0 |
| 1958 | Kanada | Vogelfanger | NRC-Stapler | Stapler | End-to-end End-to-side | 180 | 1,5 |
| 1962 | USA | Cooper | | Stapler | End-to-end | 180 | 1,5 |
| 1962 | Japan | Naka-yama | | Ring-pin | End-to-end (End-to-side) | 90 | 1,5 |
| 1983 | USA/Kanada | Daniel | Ethicon vascular coupler | Cuff-coupler | End-to-end | 180 | 1,0 |
| 1983 | Schweden | Östrup Berggren | UNILINK | Ring-pin | End-to-end End-to-side | 90 | <1,0 |

In der PCT-Anmeldung WO 87/04915 wird eine resorbierbare Vorrichtung zur makro- und mikrochirurgischen Verbindung von Gefäßenden beschrieben, bei der auf die Außenfläche einer Ringmanschette erst das umgestülpte Ende des einen Blutgefäßes und dann das aufgeweitete Ende des anderen Blutgefäßes aufgezogen wird. Die Fixierung der beiden Gefäßenden auf der Manschette erfolgt mittels eines Clips, der allerdings in der aufgezeigten Form einen erheblichen Platzbedarf besitzt. Außerdem bietet die konstruktive Ausführung des Clips keine oder nur mangelhafte Anpassungsmöglichkeiten an unterschiedliche Gefäßwandstärken. Bei Gefäßenden, die unter Längsspannung stehen, besteht die Gefahr des Herausrutschens des übergezogenen Gefäßteiles.

EP 0 154 103 beansprucht eine resorbierbare Vorrichtung zur Anastomose, bestehend aus einem Mittelstück und zwei Endstücken. Das Mittelstück ist rohrförmig ausgeführt und besitzt beidseitig aufbiegbare Finger mit Kralle. Die Gefäßenden werden durch die Endstücke geführt, umgestülpt und auf diese aufgezogen. Durch Einschieben der Endstücke in das Mittelstück erfolgt der Berührungskontakt der beiden Gefäßenden als Voraussetzung für Dichtheit des Systems und Gewebeverwachsung. Der Nachteil dieser Lösung liegt in der komplizierten konstruktiven Gestaltung des Mittelstückes, die Grenzen in Richtung kleinerer Gefäßdurchmesser setzt. Ein weiterer Nachteil wird in der schwierigen Lösbarkeit der Verbindung bei eventuellen Undichtigkeiten gesehen, und der Verbindung unterschiedlicher Gefäßdurchmesser sind Grenzen gesetzt. Das Mittelstück besitzt keine Dichtfunktion, die Dichtheit wird nur durch den Kontakt der Stoßflächen beider Endstücke gewährleistet. Dies geht zulasten der Sicherheit der Abdichtung.

In der EP 0 158 316 werden eine Anastomose-Vorrichtung, Applikationsinstrumentarien sowie ein Anastomose-Verfahren beschrieben. Die Vorrichtung besteht aus nichtresorbierbaren, biokompatiblen Materialien und wird von einem rohrförmigen Mittelstück und zwei in radialer Richtung federnden Ringe gebildet. Die Anwendung erfolgt in der Weise, daß das eine Gefäßende durch das Mittelstück durchgezogen, umgestülpt und durch den ersten Ring fixiert wird. Das zweite Gefäßende wird aufgeweitet, über das umgestülpte Gefäßende gezogen und mit dem zweiten Ring fixiert. Als Nachteil dieser Lösung stellt sich insbesondere die Tatsache dar, daß Fremdmaterial im Körper verbleibt. Weiterhin bildet sich zwischen dem übergezogenen zweiten Gefäßende und der Vorrichtung ein Gebiet deutlicher Durchmessererweiterung mit schlechter Durchströmung, was zur Thrombusbildung Anlaß geben kann.

Eine resorbierbare Vorrichtung zur Verbindung größerer Gewebehohlräume, die von außen zugänglich sind, wird in EP 0 517 488 beschrieben. Da die Vorrichtung im Inneren des Hohlraumes liegt, ist ihre Anwendung auf Blutgefäße durch Thrombosegefahr und die Querschnittsverringerung auszuschließen.

US-PS-4,470,415 beschreibt eine Anastomosevorrichtung, aufgebaut aus einem schrumpfförmigen, rohrförmigen Hohlkörper und Trägerhülsen für die Gewebeenden, wobei die Trägerhülsen aus resorbierbaren, biokompatiblen Werkstoffen bestehen. Als nachteilig ist dabei das Verbleiben eines Fremdkörpers im Organismus anzugeben.

Die Nachteile des Standes der Technik stellen sich bei den bekannten technischen Lösungen vor allem in einer komplizierten konstruktiven Gestaltung der Vorrichtung dar, die damit einen Kostennachteil aufweisen. Ein weiterer Mangel ist die nicht ausreichende Flexibilität, sich unterschiedlichen Gefäßdurchmessern anzupassen. Da die Abdichtung jeweils nur an einer Dichtfläche, entweder axial oder radial erfolgt, wird diesbezüglich noch nicht das mögliche Optimum erreicht. Einige Lösungen beanspruchen erheblichen Platz und/oder führen bei der Verbindung von Blutgefäßen zu Gebieten geringer Durchströmung mit der Gefahr der Thrombusbildung.

Aufgabe der Erfindung ist es, die Anastomosevorrichtung so zu vereinfachen, daß eine kostengünstigere Fertigung der Einzelteile der Vorrichtung möglich ist. Durch günstige Handhabung soll die Zeit zur Durchführung der Anastomose verkürzt, die Sicherheit der Abdichtung erhöht und durch Einsatz von resorbierbaren Polymeren der Verbleib von Fremdkörpern im Körper vermieden werden. Der Spielraum zur Verknüpfung von Gefäßen unterschiedlichen Durchmessers soll vergrößert werden, der Plazbedarf für die Vorrichtung klein sein. Außerdem sollen Komplikationen bei der Anwendung vermieden werden.

Erfindungsgemäß wird die Aufgabe durch die Schaffung einer Anastomosevorrichtung mit den in Anspruch 1 angegebenen Merkmalen gelöst. Vorteilhafte Ausgestaltungen sind in den Unteransprüchen 2 bis 14 angegeben.

Alle Einzelteile der erfindungsgemäßen Anastomosevorrichtung bestehen aus resorbierbaren, biokompatiblen Werkstoffen, wie z.B. aus Polylactiden, Polyglycoliden, geeigneten Copolymeren oder deren Komposite mit resorbierbaren anorganischen Biomaterialien wie Tricalciumphosphat oder Calciumdiphosphat bis zu einem Gehalt von 35 Vol-%. Als Material für den schrumpffähigen, rohrförmigen Hohlkörper kommen bevorzugt resorbierbare Polymere, wie Poly-(DL-Lactid-co-Trimethylenecarbonat) im Verhältnis 50:50 bis 90:10 (Mol-%) oder Poly-(L-Lactid-co-DL-Lactid) im Verhältnis 70:30 (Mol-%) zum Einsatz. Diese enthalten gegebenenfalls als weiteren Bestandteil anorganische, resorbierbare Biomaterialien, wie Tricalciumphosphat, Calciumdiphosphat oder schnell resorbierbare Glaskeramiken in feindisperser Verteilung bis maximal 20 Vol-%.

Der schrumpffähige, rohrförmige Hohlkörper ist aus zylindrischen Segmenten aufgebaut. Zur Verbindung mehrerer Gewebeenden sind erfindungsgemäß mehrere zylindrische Segmente des schrumpffähigen, rohrförmigen Hohlkörpers zu Y-, T-Stücken oder ähnlichen Formen kombiniert. Die zur Anwendung kommende Gestalt des schrumpffähigen, rohrförmigen Hohlkörpers und der Trägerhülsen richtet sich im einzelnen nach der zu lösenden Anastomose-Aufgabe. Eine spezielle Ausführungsform des schrumpffähigen, rohrfömigen Hohlkörpers weist für eine bessere Handhabung während der Operation eine oder mehrere Haltelaschen auf.

Auswahl und Einsatz des schrumpffähigen, rohrförmigen Hohlkörpers erfolgen nach dem Außendurchmesser und der Art der Gewebeenden, ebenso das Maß der Aufweitung des schrumpffähigen, rohrförmigen Hohlkörpers in radialer und axialer Richtung. Erfindungsgemäß liegt das Ausgangsmaß des Innendurchmessers des schrumpffähigen, rohrförmigen Hohlkörpers vor der Aufweitung beim 0,4- bis 0,8-fachen des Gewebeaußendurchmessers. Nach der Aufweitung beträgt das Maß das 2- bis 5-fache des Gewebeaußendurchmessers.

Speziell für die Anastomose von Blutgefäßen unterschiedlichen Durchmessers ist es vorteilhaft, diese mittels nur einer Trägerhülse so durchzuführen, daß das Gefäß mit dem kleineren Durchmesser in bekannter Weise durch die Trägerhülse durchgezogen und umgestülpt ist, während das Gefäß mit dem größeren Durchmesser auf die Trägerhülse bis auf die Trägerfläche größeren Durchmessers aufgezogen ist. Die Abdichtung erfolgt mittels des schrumpffähigen, rohrförmigen Hohlkörpers, der die beiden Gewebeenden fest aufeinander preßt.

Durch die Maßveränderung des schrumpffähigen, rohrförmigen Hohlkörpers beim Schrumpfungsvorgang ist es ohne Probleme möglich, z.B. bei der Verwendung von zwei Trägerhülsen, Trägerhülsen unterschiedlichen Durchmessers zur Anastomose von Gefäßen ungleicher Durchmesser einzusetzen. Auch in diesem Falle umschließt der geschrumpfte Hohlkörper beide Gefäßenden und Trägerhülsen fest in radialer Richtung und gewährleistet so eine sichere Abdichtung. Zur Realisierung der gewünschten axialen Schrumpfung ist der schrumpffähige Hohlkörper in axialer Richtung auf das 1.02- bis 1,2-fache seiner Ausgangslänge aufgeweitet.

Die Trägerhülse besitzt auf ihrer Außenoberfläche im Durchmesser abgestufte Trägerflächen für die Aufnahme der Gefäßenden und/oder des geschrumpften, rohrförmigen Hohlkörpers. Auf diesen Trägerflächen sind zur Fixierung der Gefäßenden bevorzugt spitze oder andere zur Fixierung und Kraftübertragung geeignete, erhabene Elemente angebracht. Diese übertragen gleichzeitig bei Nutzung mehrerer Trägerhülsen die bei der Schrumpfung des schrumpffähigen, rohrförmigen Hohlkörpers in axialer Richtung auftretenden Kräfte auf diese und pressen die Gefäßenden somit fest auf- bzw. gegeneinander. Bei Verwendung von mehreren Trägerhülsen zur Verbindung von mehr als zwei Gewebeenden sind die Trägerhülsen mindestens an einer Stirnseite so profiliert, daß sie nach Durchziehen und Umstülpen der Gewebeenden paßfähig sind und nach dem Schrumpfvorgang mittels des aus mehreren Segmenten bestehenden Hohlkörpers eine dichte Verbindung der Stirnseite erreicht ist.

Das für den schrumpffähigen, rohrförmigen Hohlkörper eingesetzte Material weist zur Vermeidung von Gewebeschädigungen eine Schrumpftemperatur unterhalb der kritischen Temperatur von 48 °C auf. Die obengenannten Werkstoffe tragen dieser Forderung in hervorragender Weise Rechnung, so daß Gewebebeschädigungen durch Temperaturbelastung auszuschließen sind. Zur Minderung der Gefahr von Spannungsrelaxation bei Polymeren mit Schrumpftemperaturen nahe der Körpertemperatur erhält das Material für den schrumpffähigen, rohrförmigen Hohlkörper vorteilhafterweise als weiteren Bestandteil ein anorganisches, resorbierbares Biomaterial, wie Tricalciumphosphat, Calciumdiphosphat oder schnell resorbierbare Glaskeramiken in feindisperser Verteilung bis maximal 20 Vol-%.

In einer speziellen Ausführungsform enthalten der schrumpffähige, rohrförmige Hohlkörper und die Trägerhülsen als weitere Bestandteile Wirkstoffe oder Wirkstoffkombinationen oder sind mit diesen erfindungsgemäß beschichtet. Diese Wirkstoffe stammen je nach Zielstellung aus unterschiedlichen Wirkstoffgruppen. Zur Minderung der Gefahr einer Trombusbildung hat sich z.B. die Beschichtung mit gerinnungshemmenden Wirkstoffen bzw. Wirkstoffkombinationen als vorteilhaft erwiesen.

Bezogen auf den Stand der Technik zeichnet sich die Erfindung durch eine einfache Handhabung und konstruktive Gestaltung aus und läßt damit eine besonders kostengünstige Ausführung von Anastomosen bei gleichzeitiger Erhöhung der Sicherheit der Gewebeverbindung zu. Dies macht sich vor allem bei der Anastomose von Blutgefäßen vorteilhaft bemerkbar.

Neben der Erhöhung der Sicherheit wird durch die erfindungsgemäße Lösung aufgrund ihrer einfachen und sicheren Handhabung die Operationszeit verkürzt, und die Möglichkeit zur Verbindung von Gewebeenden unterschiedlichen Durchmessers werden vergrößert.

Bedingt durch die Resorbierbarkeit aller Vorrichtungsteile verbleiben keine Fremdkörper im Organismus, so daß Zweitoperationen entfallen. Die spezielle Gestaltung der Anastomosevorrichtung gestattet, auf aufwendige Instrumentarien bei der Anwendung zu verzichten. Durch die Möglichkeit der gezielten Freisetzung von Wirkstoffen wird die Gefahr von operativ bedingten Komplikationen vermindert.

Die Erfindung wird nachstehend anhand von Zeichnungen und Ausführungsbeispielen erläutert.

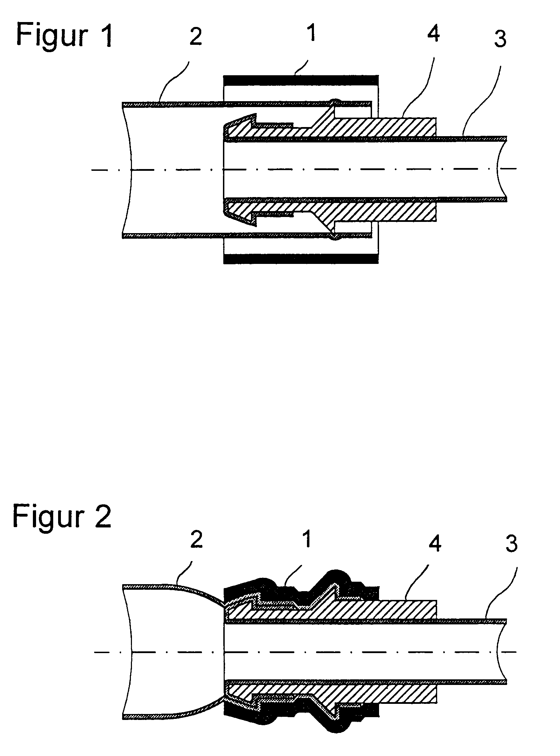

Figur 1 zeigt die Anastomosevorrichtung in Schnittdarstellung bei Nutzung eines schrumpffähigen, rohrförmigen Hohlkörpers 1 und einer Trägerhülse 4 mit positionierten Gefäßen 2,3 unmittelbar vor dem Schrumpfvorgang
und Figur 2 nach der Schrumpfung

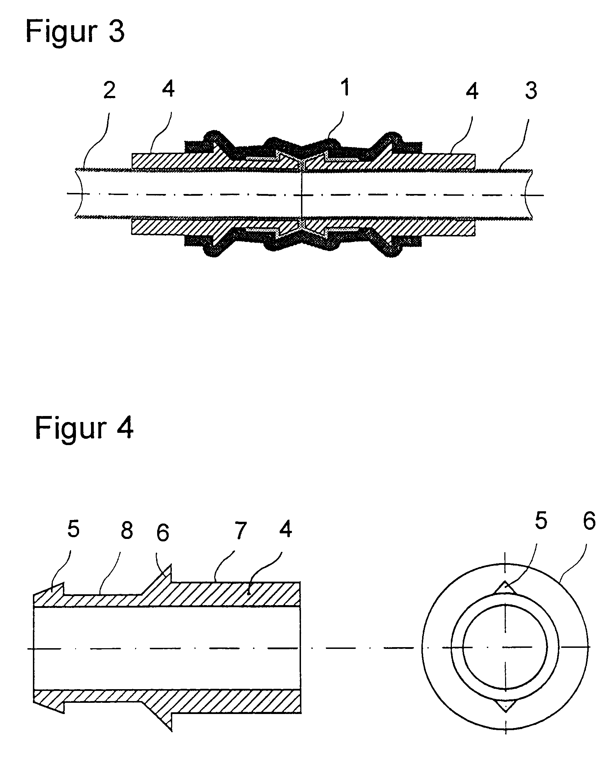

Figur 3 stellt einen Schnitt der Anastomosevorrichtung bei Nutzung eines rohrförmigen, schrumpffähigen Hohlkörpers 1 und zweier Trägerhülsen 4 nach der Schrumpfung dar.
Figur 4 enthält die Schnittdarstellung und Vorderansicht einer Trägerhülse 4 mit verschiedenen zur Fixierung und Kraftübertragung geeigneten erhabenen Elementen 5 und 6.

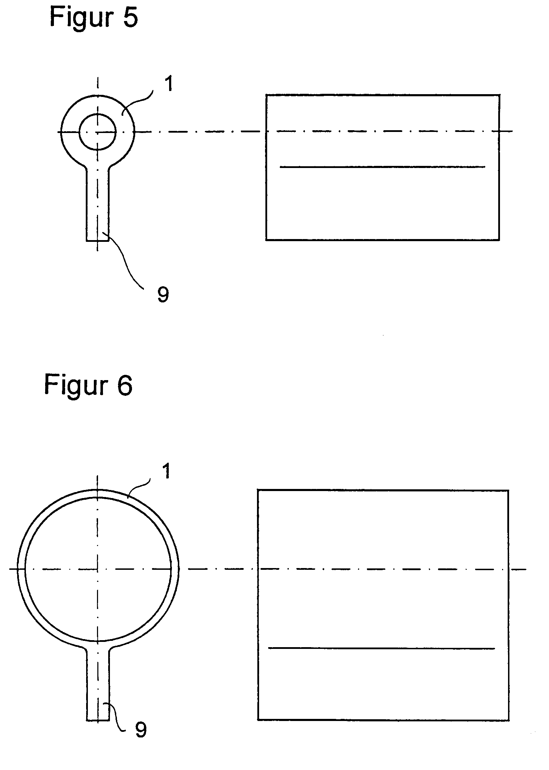

Figur 5 und 6 zeigen den rohrförmigen, schrumpffähigen Hohlkörper 1 in seinem unaufgeweiteten und aufgeweiteten Zustand mit Haltelasche 9 in Schnittdarstellung.

### Ausführungsbeispiel 1:

Ein schrumpffähiger, rohrförmiger Hohlkörper 1, aus drei zylindrischen Segmenten zu einem T-Stück aufgebaut, gefertigt aus einem Copolymeren der Zusammensetzung (in Mol-%) 90 DL-Lactid und 10 Trimethylencarbonat, mit einem Innendurchmesser von 6 mm vor und 12 mm nach der Aufweitung, wird mit drei stirnseitig paßfähigen Trägerhülsen 4 aus dem gleichen Material kombiniert. Die Länge eines zylindrischen Segmentes vor der Aufweitung betrug 12 mm und nach der Aufweitung 13 mm. Nachfolgend wurde der schrumpffähige, rohrförmige Hohlkörper 1 unter seine Glasübergangstemperatur von 35 ° C abgekühlt.

Die Gewebeenden wurden durch die Trägerhülsen 4 gezogen, auf die Trägerfläche 8 umgestülpt und mittels Elementen 5 fixiert. Anschließend wurden die Trägerhülsen 4 paßfähig in den aufgeweiteten schrumpffähigen, rohrförmigen Hohlkörper 1 positioniert und dieser durch Aufträufeln von physiologischer Kochsalzlösung mit einer Temperatur von 40° C geschrumpft.

Die Gewebeenden sind fest gegeneinander gepreßt und der T-förmige schrumpffähige Hohlkörper 1 umschließt alle Trägerhülsen und Gewebeenden dicht und fest.

### Ausführungsbeispiel 2:

Ein schrumpffähiger, rohrförmiger Hohlkörper 1, gefertigt aus einem Copolmeren der Zusammensetzung (in Mol-%) 70 L-Lactid und 30 DL-Lactid, mit einem Innendurchmesser von 3,5 mm vor und 9 mm nach der Aufweitung wird mit einer Trägerhülse 4 aus dem gleichen Material kombiniert. Die Länge des schrumpffähigen, rohrförmigen Hohlkörpers 1 vor der Aufweitung betrug 10 mm und nach der Aufweitung 11,5 mm. Nach der Aufweitung wurde der schrumpffähige, rohrförmige Hohlkörper 1 unter seine Glasübergangstemperatur von 55° C abgekühlt.

Das Gewebeende 3 mit kleinerem Durchmesser wurde durch die Trägerhülse 4 gezogen, auf die Trägerfläche 8 umgestülpt und mittels der Elemente 5 fixiert. Das Gewebeende mit dem größeren Durchmesser wurde über das auf der Trägerfläche 8 liegende Gefäßende auf die Trägerfläche 7 geschoben und mittels Element 6 fixiert. Der schrumpffähige, rohrförmige Hohlkörper 1 wurde über der Trägerhülse 4 mit den aufgezogenen Gewebeenden positioniert und auf eine Temperatur von 60° C zur Schrumpfung erwärmt.

Die Gewebeenden sind fest gegeneinander gepreßt und der schrumpffähige, rohrförmige Hohlkörper 1 umschließt die Trägerhülse 4 mit den Gewebeenden 2 und 3 dicht und fest.

Weitere Ausführungsbeispiele sind in der Tabelle 2 dargestellt.

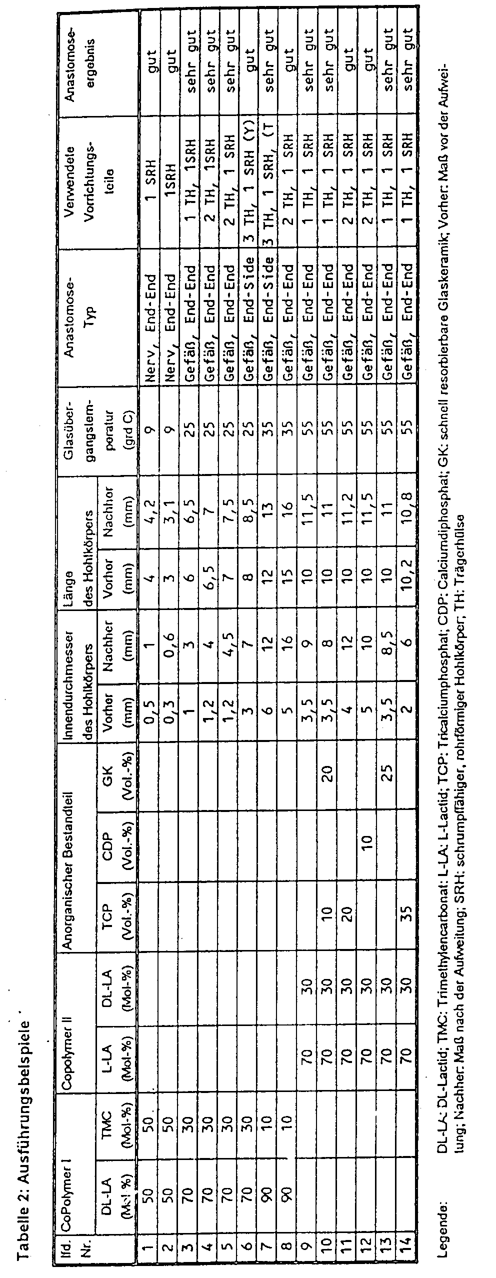

### Aufstellung der verwendeten Bezugszeichen

- 1: Schrumpffähiger, rohrförmiger Hohlkörper
- 2: Gewebeende, z.B. Blutgefäß
- 3: Gewebeende, z. B. Blutgefäß
- 4: Trägerhülse
- 5: Fixierendes Element
- 6: Fixierendes Element
- 7: Trägerfläche
- 8: Trägerfläche
- 9: Haltelasche

## Patentansprüche

1. Anastomosevorrichtung, bestehend aus wenigstens einer Trägerhülsen aus resorbierbarem biokompatiblem Werkstoff für die Gewebeenden und aus einem diese verbindenden schrumpffähigen, rohrförmigen Hohlkörper aus biokompatiblem Werkstoff, **dadurch gekennzeichnet**, **daß** der schrumpffähige rohrförmige Hohlkörper (1) aus resorbierbaren Polymeren aus der Gruppe der Polylactide oder Polyglycoliden besteht.

2. Anastomosevorrichtung, nach Anspruch 1, **dadurch gekennzeichnet**, **daß** der schrumpffähige rohrförmige Hohlkörper (1) aus resorbierbaren Copolymeren des Poly-(L-Lactid-co-D,L-Lactid) im Verhältnis 70:30 in Mol-% besteht.

3. Anastomosevorrichtung, nach Anspruch 1 oder 2, **dadurch gekennzeichnet, daß** der schrumpffähige rohrförmige Kohlkörper (1) aus Copolymeren des Poly-(DL-Lactid-co-Trimethylenecarbonat) im Verhältnis 50 : 50 bis 90:10 in Mol-% besteht.

4. Anastomosevorrichtung nach einem der Anspruch 1 bis 3, **dadurch gekennzeichnet, daß** der schrumpffähige, rohrförmige Hohlkörper (1) zusätzlich anorganische, die Schrumpfungstemperatur und die Resorptionseigenschaften modifizierende, resorbierbare Biomaterialien bis 20 Vol-% enthält.

5. Anastomosevorrichtung nach einem der vorgehenden Ansprüche, **dadurch gekennzeichnet, daß** mindestens eine Trägerhülse (4) aus Copolymeren des Poly-(L-Lactid-co-D,L-Lactid) im Verhältnis 70:30 in Mol-% besteht und zusätzlich anorganische, resorbierbare Biomaterialien bis 35 Vol-% zur Modifizierung dar Resorptionseigenschaften enthält.

6. Anastomosevorrichtung nach einem der vorgehenden Ansprüche, **dadurch gekennzeichnet, daß** der schrumpffähige, rohrförmige Hohlkörper (1) aus mindestens einem zylindrischen Segment aufgebaut ist.

7. Anastomosevorrichtung nach einem der vorgehenden Ansprüche, **dadurch gekennzeichnet, daß** der zylindrische Innendurchmesser des schrumpffähigen, rohrförmigen Hohlkörpers (1) vor dem Aufweiten auf das Maß vor der Schrumpfung das 0,4- bis 0,8-fache des Gewebeaußendurchmessers beträgt.

8. Anastomosevorrichtung nach einem der vorgehenden Ansprüche, **dadurch gekennzeichnet, daß** der zylindrische Innendurchmesser des schrumpffähigen, rohrförmigen Hohlkörpers (1) vor dem Schrumpfungsvorgang das 2- bis 5-fache des Gewebeaußendurchmessers beträgt.

9. Anastomosevorrichtung nach einem der vorgehenden Ansprüche, **dadurch gekennzeichnet, daß** die Längsausdehnung des schrumpffähigen, rohrförmigen Hohlkörpers (1) vor der Schrumpfung das 1,02- bis 1,2-fache der unaufgeweiteten Ausgangslänge beträgt.

10. Anastomosevorrichtung nach einem der vorgehenden Ansprüche, **dadurch gekennzeichnet, daß** der schrumpffähige, rohrförmige Hohlkörper (1) mindestens eine Haltelasche (9) aufweist.

11. Anastomosevorrichtung nach einem der vorgehenden Ansprüche, **dadurch gekennzeichnet, daß** mindestens eine Trägerhülse (4) auf ihrer Außenoberfläche mindestens zwei in ihren Durchmessern abgestufte Trägerflächen (7,8) zur Aufnahme der Gewebeenden (2,3) und/oder des geschrumpften, rohrförmigen Hohlkörpers 1 mit zur Fixierung und Kraftübertragung geeigneten Elementen (5,6) aufweist.

12. Anastomosevorrichtung nach einem der vorgehenden Ansprüche, **dadurch gekennzeichnet, daß** die Trägerhülsen (4) bei der Verbindung von mehr als zwei Gewebeenden für eine dichte Paßfähigkeit stirnseitig gleich oder unterschiedlich profiliert ausgebildet sind.

13. Anastomosevorrichtung nach einem der vorgehenden Ansprüche, **dadurch gekennzeichnet, daß** der schrumpffähige, rohrförmige Hohlkörper (1) und die Trägerhülsen (4) zusätzlich Wirkstoffe aus der Gruppe des gerinnungshemmenden Stoffe enthalten.

14. Anastomosevorrichtung nach einem der vorgehenden Ansprüche, **dadurch gekennzeichnet, daß** der schrumpffähige, rohrförmige Hohlkörper (1) und die Trägerhülsen (4) an ihren Oberflächen beschichtet sind und diese Oberflächenschicht Wirkstoffe aus dar Gruppe der gerinnungshemmenden Stoffe enthält.

## Claims

1. Anastomosis device constructed of a shrinkable, tubular hollow body and support sleeves for the ends of tissue, where the inner diameters of the anastomosis device is adapted to the outer diameters of the tissue,
wherein
the anastomosis device is formed by a shrinkable, tubular hollow body (1) and preferably a special support sleeve (4), and wherein the shrinkable, tubular hollow body (1) and the support sleeve (4) are composed of the same or different absorbable, biologically compatible materials.

2. Device according to claim 1, wherein the absorbable, biologically compatible material is a copolymer of the type poly-(L-lactide-co-DL-lactide) in a ratio of 70:30 (in mol-%).

3. Device according to claim 1, wherein the shrinkable, tubular hollow body (1) is composed of copolymers of the poly-(DL-lactide-co-trimethyelene carbonate) in a ratio of 50:50 to 90:10 (in mol-%) or poly-(L-lactide-co-D,L-lactide) in a ratio of 70:30 (in mol-%), and possibly additional inorganic, absorbable biologically compatible materials of up to 20 vol-% are contained for modifying the shrinking temperature and/or for modifying the absorptive properties.

4. Device according to claim 1, wherein the support sleeve (4) is composed of copolymers of the poly-(DL-lactide-co-trimethylene carbonate) in a ratio of 50:50 to 90:10 (in mol-%) or poly-(L-lactide-co-D,L-lactide) in a ratio of 70:30, and possibly additional inorganic, absorbable biologically compatible materials of up to 35 vol-% are contained for modifying the absorptive properties.

5. Device according to claim 1, wherein the shrinkable, tubular hollow body (1) is constructed of a cylindrical segment or of several cylindrical segments, preferably in a Y shape or a T shape.

6. Device according to claim 1, wherein the cylindrical inner diameter of the shrinkable, tubular hollow body (1) amounts to 0.4 times to 0.8 times the outer diameter of the tissue prior to the expansion of the size prior to shrinking.

7. Device according to claim 1, wherein the cylindrical inner diameter of the shrinkable, tubular hollow body (1) amounts to 2 times to 5 times the outer diameter of the tissue prior to the shrinking process.

8. Device according to claim 1, wherein the longitudinal extension of the shrinkable, tubular hollow body (1) amounts to 1.02 to 1.2 times the non-expanded starting length prior to shrinking.

9. Device according to claim 1 and claims 5 to 8, wherein the shrinkable, tubular hollow body (1) exhibits possibly one or several support brackets (9).

10. Device according to claim 1, wherein the support sleeve (4) exhibits on its outer face at least two support faces (7, 8), stepped in their diameters, for holding the ends (2, 3) of the tissue and/or of the shrunk, tubular hollow body (1) with elements (5, 6) suitable for fixing and for force transmission.

11. Device according to claim 1, wherein the support sleeves (4) are formed on the front face with a uniform or non-uniform profile upon connection of more than two ends of the tissue for a capability of fitting and sealing.

12. Device according to claims 1, 2, and 3, wherein the shrinkable, tubular hollow body (1) and the support sleeve (4) contain in addition suitable effective materials or combinations of effective materials, preferably of the group of anticoagulative materials.

13. Device according to claim 1, wherein the shrinkable, tubular hollow body (1) and the support sleeve (4) are coated at their surfaces, and wherein this surface layer contains suitable effective materials or combinations of effective materials, preferably of the group of anticoagulative materials.

14. Anastomosis device as claimed in any of the precending claims, characterized in that the shrinkable tubular hollow body (1) and the support sleeves (4) are coated at their surfaces, and wherein this surface layer contains active agents of the group of anticoagulants.

## Revendications

1. Dispositif d'anastomose consistant d'au moins une douille de support d'un matériau résorbable biocompatible pour les bouts de tissu et d'une pièce creuse tubulaire rétractable d'un matériau biocompatible, cette pièce creuse reliant les bouts de tissu, caractérisé en ce que la pièce creuse tubulaire rétractable (1) consiste de polymères résorbables du groupe des polylactides ou polyglycolides.

2. Dispositif d'anastomose selon la revendication 1, caractérisé en ce que la pièce creuse tubulaire rétractable (1) consiste de copolymères résorbables du poly-(L-lactide-co-D-, L-lactide) au prorata de 70 :30 en mol%.

3. Dispositif d'anastomose selon l'une quelconque des revendications 1 et 2, caractérisé en ce que la pièce creuse tubulaire rétractable (1) consiste de copolymères du poly-(DL-lactide-co-carbonate de triméthylène) au prorata de 50:50 jusqu'à 90:10 en mol%.

4. Dispositif d'anastomose selon l'une quelconque des revendications 1, 2 et 3, caractérisé en ce que la pièce creuse tubulaire rétractable (1) contient en plus des matériaux biologiques inorganiques résorbables jusqu'à 20 vol%, ces matériaux inorganiques modifiant la température de contraction et les propriétés de résorption.

5. Dispositif d'ananastomose selon l'une quelconque des revendications précédentes, caractérisé en ce que au moins une douille de support (4) consiste de copolymères du poly-(L-lactide-co-D-, L-lactide) au prorata de 70:30 en mol% et en plus des matériaux biologiques inorganiques résorbables jusqu'à 35 vol% servant à modifier les propriétés de résorption.

6. Dispositif d'anastomose selon l'une quelconque des revendications précédentes, caractérisé en ce que la pièce creuse tubulaire rétractable (1) consiste d'au moins un segment cylindrique.

7. Dispositif d'anastomose selon l'une quelconque des revendications précédentes, caractérisé en ce que le diamètre intérieur cylindrique de la pièce creuse tubulaire rétractable (1) est de 0,4 à 0,8 fois le diamètre extérieur du tissu avant l'élargissement jusqu'à la dimension avant la contraction.

8. Dispositif d'anastomose selon l'une quelconque des revendications précédentes, caractérisé en ce que le diamètre intérieur cylindrique de la pièce creuse tubulaire rétractable (1) est de 2 à 5 fois le diamètre extérieur du tissu avant le processus de contraction.

9. Dispositif d'anastomose selon l'une quelconque des revendications précédentes, caractérisé en ce que l'étendue en longueur de la pièce creuse tubulaire rétractable (1) est de 1,02 à 1,2 fois la longueur initiale avant l'élargissement.

10. Dispositif d'anastomose selon l'une quelconque des revendications précédentes, caractérisé en ce que la pièce creuse tubulaire rétractable (1) est munie d'au moins un collier de fixation (9).

11. Dispositif d'anastomose selon l'une quelconque des revendications précédentes, caractérisé en ce que au moins une douille de support (4) a sur sa surface extérieure au moins deux plats de support (7,8) à diamètres gradués servant à la réception des bouts de tissu (2,3) et/ou de la pièce creuse tubulaire rétractée (1), ces plats de support étant munis d'éléments de fixation et de transmission de force (5,6) appropriés.

12. Dispositif d'anastomose selon l'une quelconque des revendications précédentes, caractérisé en ce que les douilles de support (4) présentent des profils identiques ou différents sur la face pour la liaison de plus de deux bouts de tissu de sorte à assurer l'étanchéité absolue.

13. Dispositif d'anastomose selon l'une quelconque des revendications précédentes, caractérisé en ce que la pièce creuse tubulaire rétractable (1) et les douilles de support (4) contiennent en plus des matières actives du groupe des anticoagulants.

14. Dispositif d'anastomose selon l'une quelconque des revendications précédentes, caractérisé en ce que la pièce creuse tubulaire rétractable (1) et les douilles de support (4) sont munies d'une couche superficielle contenant des matières actives du groupe des anticoagulants.
